# EUROPÄISCHE PATENTSCHRIFT

(11) **EP 1 894 516 B1**
(45) Veröffentlichungstag und Bekanntmachung des Hinweises auf die Patenterteilung: **06.04.2016**
(21) Anmeldenummer: 07016792.9
(22) Anmeldetag: 28.08.2007
(51) Int. Cl.: A61B 1/07

(54) **Beleuchtungssystem zum Erzeugen von Licht und zum Einkoppeln des Lichts in ein proximales Ende eines Lichtleitkabels einer Beobachtungsvorrichtung für die Endoskopie oder Mikroskopie**
Illumination system for creating light and for injecting light into the proximal end of a light conducting cable of an observation device for endoscopy or microscopy
Système d'éclairage pour la production de lumière et l'introduction de la lumière dans une extrémité proximale d'un câble à fibres optiques d'un dispositif d'observation pour l'endoscopie ou la microscopie

(30) Priorität: 30.08.2006 DE 102006041959
(43) Veröffentlichungstag der Anmeldung: 05.03.2008
(73) Patentinhaber: Karl Storz GmbH & Co. KG, 78532 Tuttlingen (DE)
(72) Erfinder: Ehrhardt, André, 78573 Wurmlingen (DE); Irion, Klaus, Dr.-Ing., 78576 Emmingen-Liptingen (DE); Martin, Uwe, 78549 Spaichingen (DE); Lei, Fang, Dr.-Ing., 78591 Durchhausen (DE)
(74) Vertreter: Witte, Weller & Partner Patentanwälte mbB

(56) Entgegenhaltungen:
- WO-A-2005/035059
- JP-A- 2005 347 223
- US-A1- 2004 127 961
- US-A1- 2004 260 365
- US-A1- 2005 201 100
- US-A1- 2006 171 693

## Beschreibung

Die Erfindung betrifft ein Beleuchtungssystem zum Erzeugen von Licht und zum Einkoppeln des Lichts in ein proximales Ende eines Lichtleitkabels einer Beobachtungsvorrichtung für die Endoskopie oder Mikroskopie, mit einem Lichtleitkabel, mit einer zumindest eine LED aufweisenden Lichtquelle, mit einem als Glaskörper ausgebildeten oder aus Lichtleitfasern aufgebauten und elektrisch isolierenden optischen Lichtleitelement zwischen der Lichtquelle und dem proximalen Ende des Lichtleitkabels zum Einleiten des von der lichtquelle emittierten Lichts in das proximale Ende des Lichtleitkabels, wobei das Lichtleitkabel zwischen dem Lichtleitelement und einer mit dem Lichtleitkabel zu verbindenen Beobachtungsvorrichtung angeordnet ist, wobei ein erster Endbereich des Lichtleitelements auf einer der Lichtquelle zugewandten Seite eine kleinere Querschnittsfläche als ein zweiter Endbereich des optischen Lichtleitelements auf einer dem proximalen Ende des Lichtleitkabels zugewandten Seite aufweist, und mit einer Kühlvorrichtung, die zumindest einen Kühlkörper aufweist und die mit der Lichtquelle thermisch leitend verbunden ist, um von der Lichtquelle erzeugte Wärme aus dem Beleuchtungssystem abzuführen.

Ein derartiges Beleuchtungssystem ist aus der US 2005/0201100 A1 bekannt.

Unter einer thermisch leitenden Verbindung ist im Rahmen der vorliegenden Erfindung ein direkter thermischer Kontakt oder ein indirekter thermischer Kontakt zwischen der Kühlvorrichtung, d. h. dem Kühlkörper, und der Lichtquelle zu verstehen, über den die von der Lichtquelle erzeugte Wärme wirksam abgeführt werden kann.

Ein solches Beleuchtungssystem wird in der Endoskopie oder Mikroskopie verwendet, um einen zu beobachtenden Bereich auszuleuchten. Das Beleuchtungssystem wird im Allgemeinen mittels eines Lichtleitkabels an ein Endoskop oder ein Mikroskop angeschlossen, in denen die Lichtleitung über eine Lichtleitoptik in Form von optischen Elementen und/oder Lichtleitern erfolgt. Das Beleuchtungssystem muss folglich eine ausreichende Lichtleistung bereitstellen, um den zu beobachtenden Bereich optimal ausleuchten zu können. Aufgrund der hohen Lichtleistung produziert die Lichtquelle eine hohe Wärmemenge. Diese von der Lichtquelle erzeugte Wärmemenge muss abgeführt werden, um die Leistung der Lichtquelle nicht zu beeinträchtigen.

Aus der oben genannten US 2005/0201100 A1 list ein Beleuchtungssystem bekannt, das eine LED als Lichtquelle aufweist. Zwischen der LED und einem Lichtleitkabel, mit dem das Licht zum zu beobachtenden Bereich hingeleitet wird, ist ein optisches Lichtleitelement in Form einer Linse angeordnet. Die Linse dient zum Einkoppeln des Lichts in das Lichtleitkabel, damit Intensitätsverluste an der Einkoppelstelle zwischen der LED und dem Lichtleitkabel reduziert werden. Ein erster Endbereich der Linse an der zur LED gewandten Seite der Linse weist eine kleinere Querschnittsfläche als ein zweiter Endbereich der Linse auf, der zum Lichtleitkabel hingewandt ist.

Ferner weist das bekannte Beleuchtungssystem eine Kühlvorrichtung auf, die einen zylinderförmigen Kühlkörper aufweist, der von der LED beabstandet ist und der die LED und die Linse zumindest teilweise umgibt. Die von der LED erzeugte Wärme wird durch die LED-Fassung, durch eine Kühlplatte, die an einer der Linse abgewandten Seite der LED angeordnet ist, oder durch einen Reflektor, in dem die LED aufgenommen ist, zum Kühlkörper abgeführt.

Ein Nachteil dieses Beleuchtungssystems ist, dass die von der LED erzeugte Wärme mittels kleiner Bauteile, d. h. der LED-Fassung, der Kühlplatte und des Reflektors, mit denen die Linse in direktem oder indirektem thermischen Kontakt steht, abgeleitet wird. Aufgrund der großen Wärmeerzeugung der LED erwärmen sich diese kleinen Bauteile und folglich die Linse stark. Hierdurch ist eine thermische Stabilität der Linse nicht gewährleistet, so dass sich ihre optischen Eigenschaften (Brechzahl o. Ä.) ändern können. Die beeinträchtigte Funktionsweise der Linse kann zu einer Verringerung der Lichtleistung des Beleuchtungssystems führen.

Ferner erweist es sich als nachteilig, dass der Kühlkörper räumlich beabstandet von der Lichtquelle angeordnet ist. Die Wärmeleitung von der Lichtquelle zum Kühlkörper erfolgt über die oben genanten kleinen Bauteile, die Engstellen für die Wärmeleitung darstellen, so dass folglich die von der LED erzeugte Wärmemenge schlecht an den Kühlkörper abgegeben Wird. Diese nicht ausreichende Kühlung beeinträchtigt die Funktionsweise des Beleuchtungssystems.

Ein weiterer Nachteil des Beleuchtungssystems ist seine Bauweise, bei der die LED und die Linse in dem zylindrischen Kühlkörper aufgenommen sind. Muss die LED aufgrund ihrer begrenzten Lebenddauer oder die Linse infolge einer Beschädigung ausgewechselt werden, so erweisen sich diese Reparaturarbeiten als besonders zeitaufwändig, da beide Bauteile nicht frei zugänglich, sondern in dem Kühlkörper angeordnet sind.

Aus US 2004/0127961 A1 ist eine therapeutische Lichtquelle bekannt, die beispielsweise in der photodynamischen Therapie verwendet wird. Die Lichtquelle weist ein luftgekühltes Array von LED's auf, wobei die Luft in der Nähe des Arrays ventiliert. Jede LED ist auf ihrer der Lichtaustrittsseite abgewandten Seite mit ihren Leiterfüßchen auf einem jeweiligen einielnen Plättchen als Wärmesenke befestigt, und das Array aus den Wärmesenken wird mittels eines Gebläses gekühlt.

Aus WO 2005/035059 A1 ist eine tragbare Leuchte für die extrakorporale photodynamische Diagnose für medizinische und kosmetische Zwecke bekannt, die eine Lampe aufweist, die Licht in Wellenlängenbereichen emittiert, die um das Absorptionsmaximum eines photoempfindlichen Stoffes herum liegen. Die Lampe besteht aus einer großen Anzahl von LED's, die nahe beieinander und gleichmäßig in zwei Dimensionen auf einem Basiskörper beabstandet angeordnet sind, der Wärmeenergie leitet und elektrische Energie isoliert. Der Basiskörper, der als Kühlkörper dient, ist auf der der Lichtaustrittsseite der LED's abgewandten Seite der LED's angeordnet.

Ferner offenbart JP 2005/347 223 A eine Lichtquelle, die einen Lichtleiter 12 aufweist, der Licht von einem Lichtemissionskörper aufnimmt und weiterleitet, wobei zwischen dem Lichtemissionskörper und dem Lichtleiter ein sich von dem Lichtemissionskörper zum Lichtleiter hin verjüngendes Lichtleitelement aufweist.

US 2004/0260365 A1 offenbart eine Lampe für die photodynamische Therapie, die eine Lichtquelle aufweist, die aus zwei Arrays von LEDs gebildet ist. Die LEDs sind in einem Bienenwabenmuster angeordnet. Jeder der LEDs ist eine Linse zugeordnet. Weiter in Lichtausbreitungsrichtung gesehen ist den Linsen ein Diffusor nachgeschaltet. Die Linsen sind in einem Bienenwabenmuster angeordnet und dienen dazu, das Licht zu einem im Wesentlichen parallelen und schmalen Strahl zu konzentrieren.

US 2006/0171693 A1 offenbart eine LED-basierte Lichtquelleneinheit für ein endoskopisches Abbildüngssystem, das eine Beleuchtung für eine Kamera durch ein Endoskop erzeugt. Die Lichtquelleneinheit umfaßt ein Array von LEDs, das an einem wärmeleitfähigem Substrat angebracht ist. Die Lichtquelleneinheit ist in das proximale Ende eines Endoskops integriert und unmittelbar an die optischen Fasern gekoppelt, die zur Spitze des Endoskops verlaufen.

Es ist eine Aufgabe der vorliegenden Erfindung, hier Abhilfe zu schaffen und ein Beleuchtungssystem der eingangs genannten Art dahingehend weiterzubilden, dass es eine effiziente Kühlung der Lichtquelle ermöglicht, ohne dass das optische Lichtleitelement beeinträchtigt wird, und dass die Betriebssicherheit des Beleuchtungssystems erhöht ist.

Erfindungsgemäß wird die Aufgabe dadurch gelöst, dass der Kühlkörper vollständig auf einer dem optischen Lichtleitelement abgewandten Seite der Lichtquelle angeordnet ist, dass das Lichtleitelement von der Lichtquelle geringfügig beabstandet angeordnet ist und dass das Lichtleitelement in einem elektrisch isolierenden Halteelement angeordnet ist.

Das Anordnen des Kühlkörpers auf einer dem optischen Lichtleitelement abgewandten Seite der Lichtquelle ermöglicht ein Ableiten oder von der Lichtquelle erzeugten Wärme außerhalb eines Bereichs, in dem das optische Lichtleitelement angeordnet ist. Hierdurch tritt vorteilhafterweise keine thermische Wechselwirkung zwischen der Kühlvorrichtung, und dem optischen Lichtleitelement auf, so dass sich dessen optische Eigenschaften (Brechzahl o. Ä.) auch während des Betriebs des Beleuchturtgssystems nicht ändern. Auf diese Weise bleibt eine gleichbleibende Lichtleistung des von der Lichtquelle emittierten und in das Lichtleitkabel eingekoppelten Lichts erhalten.

Ein weiterer Vorteil einer vollständigen Anordnung des Kühlkörpers auf einer dem optischen Lichtleitelement abgewandten Seite der Lichtquelle ist, dass ein Ausbauschen der Lichtquelle und des optischen Lichtleitelements besonders einfach und zeitsparend durchgeführt werden kann. Da die Lichtquelle und das optische Lichtleitelement frei zugänglich und nicht in dem Kühlkörper aufgenommen sind, wird während der Reparatur keine zusätzliche Zeit zum Aus- bzw. Einbauen des Kühlkörpers benötigt.

Das optische Lichtleitelement ist elektrisch isolierend ausgebildet.

Diese Maßnahme hat den Vorteil, dass kein Sttomfluss von der mit Spannung beaufschlagbaren Lichtquelle auf das optische Lichtleitelement, d.h. auf das Lichtleitkabel und das Gehäuse, überführbar ist. Dies erhöht die Sicherheit der Person, die, das Beleuchtungssystem während bspw. medizinischen Untersuchungen verwendet. Ferner kann sich das Lichtleitkabel nicht durch den eingespeisten Strom erwärmen, so dass es nicht durch Aufheizung in seiner Funktionsweise beeinträchtigt wird.

Das optische Lichtleitelement ist als Glaskörper, insbesondere als Linse, ausgebildet.

Diese Maßnahme ermöglicht vorteilhafterweise eine ausreichend aus dem Stand der Technik bekannte Ausgestaltungsform des optischen Lichtleitelements, um das von der Lichtquelle emittierte Licht in das Lichtleitkabel einzukoppeln. Ferner ist ein. Glaskörper besonders kostengünstig herzustellen und gewährleistet aufgrund seiner geringen elektrischen Leitfähigkeit eine elektrische Isolierung zwischen der Lichtquelle und dem Lichtleitkabel. Die Linse kann bspw. als Compound Elliptical Concentrator (CEC) oder als Compound Hyperbolic Concentrator (CHC) ausgebildet sein.

In einer alternativen Ausgestaltung ist das optische Lichtleitelement aus optischen Lichtleitfasern aufgebaut.

Diese Maßnahme bietet ebenso eine vorteilhafte Ausgestaltung des optischen Lichtleitelements. Die Verwendung von Lichtleitfasern vermeidet eine aufwändige Oberflächenform des Glaskörpers zur Lichtleitung, da die von der Lichtquelle emittierten Lichtstrahlen durch die Ausrichtung der optischen Lichtleitfasern durch das optischen Lichtleitelement geleitet werden. Ferner ermöglicht die Verwendung von Lichtleitfasern ein Ausnutzen der gesamten zur Stirnseite des Lichtleitkabels hingewandten Oberfläche des zweiten Endbereichs des optischen Lichtleitelements, um die Lichtstrahlen in das Lichtleitkabel einzukoppeln.

Das optische Lichtleitelement ist von der Lichtquelle geringfügig beabstandet angeordnet.

Diese Maßnahme hat den Vorteil, dass diese geringfügig beabstandete Anordnung des Lichtleitelements von der Lichtquelle eine weitere elektrische Isolation des optischen Lichtleitelements von der Lichtquelle bereitstellt. Hierdurch wird eine Beschädigung des optischen Lichtleitelements vermieden und es verringert sich ferner eine Gefahr für die das Beleuchtungssystem verwendende Person.

Das Beleuchtungssystem weist ein elektrisch isolierendes Halteelement auf, in dem das optische Lichtleitelement angeordnet ist.

Diese Maßnahme ermöglicht vorteilhafterweise eine weitere Isolation der mit Spannung beaufschlagbaren Lichtquelle von dem optischen Lichtleitelement und dem Gehäuse des Beleuchtungssystems, so dass ebenfalls eine Personengefahr verringert wird.

In einer bevorzugten Ausgestaltung sind die Lichtquelle, das optische Lichtleitelement, das proximale Ende des Lichtleitkabels und der Kühlkörper in einem gemeinsamen Gehäuse angeordnet.

Diese Maßnahme hat den Vorteil, dass das Beleuchtungssystem eine kompakte Bauweise aufweist, die technisch einfach zu realisieren ist. Ferner erweist sich diese Ausgestaltung des Beleuchtungssystems während des Betriebs als vorteilhaft, da das Gehäuse des Beleuchtungssystems nur aus einem Gehäuseteil, nicht aus mehreren Gehäuseteilen, besteht, so dass bspw. bei Inbetriebnahme des Beleuchtungssystems keine Schwierigkeiten auftreten können, die aus einem Zusammenbauen von mehreren Gehäuseteilen resultieren.

In einer weiteren bevorzugten Ausgestaltung ist der Kühlkörper thermisch leitend, insbesondere direkt thermisch leitend, mit dem Gehäuse verbunden.

Unter einer thermisch leitenden Verbindung des Kühlkörpers mit dem Gehäuse ist ein direkter thermischer Kontakt oder ein indirekter thermischer Kontakt beider Bauteile zu verstehen. Bei einem indirekten thermische Kontakt sind bspw. zusätzliche wärmeleitende Bauteile zwischen dem Kühlkörper und dem Gehäuse angeordnet.

Diese Maßnahme hat den Vorteil, dass die Wärme nicht nur über den Kühlkörper in das Innere des Beleuchtungssystems abgegeben, sondern ebenfalls über das Gehäuse nach außen abgeleitet wird. Hierdurch entsteht kein Wärmestau innerhalb des Beleuchtungssystems, so dass die Funktionsweise des Beleuchtungssystems nicht beeinträchtigt wird.

In einer weiteren bevorzugten Ausgestaltung ist der Kühlkörper als passive Kühlung, insbesondere als Heatpipe, ausgebildet.

Diese Maßnahme hat den Vorteil, dass die Kühlung des Beleuchtungssystems über Wärmekonvektion erfolgt. Diese Art der Kühlung ist ausreichend aus dem Stand der Technik bekannt und vermeidet ferner zusätzliche Bauteile, wie z. B. Leitungen, zum Führen einer Kühlflüssigkeit oder eines Gases zur Lichtquelle: Ferner verringert eine passive Kühlung die Gefahr eines Kurzschlusses, der insbesondere durch einen Kontakt der Kühlflüssigkeit oder des Gases mit den elektrischen Zuletrungen der Lichtquelle oder der Lichtquelle selbst auftreten kann.

Die Ausgestaltung des Kühlkörpers als Heatpipe ermöglicht vorteilhafterweise einen sehr effizienten, aus dem Stand der Technik bekannten Kühlmechanismus. Hierbei ist der Kühlkörper als abgeschlossener Hohlkörper aus einem wärmeleitfähigen Material, bspw. Aluminium, ausgebildet, an dessen Innenseite ein kapillar wirksames, dochtartiges Material angeordnet ist, und der ferner mit einer Flüssigkeit unter Eigendruck oder ggf. unter reduziertem Druck befüllt ist. Wird dem Hohlkörper an seiner Oberfläche Wärme zugeführt, beginnt die in einem Inneren des Höhlkörpers befindliche Flüssigkeit zu sieden und geht unter Aufnahme von Wärmeenergie in Dampf über. Dieser Dampf verteilt sich in dem Hohlkörperinnenraum und kondensiert unter Wärmeabgabe an einer kälteren Stelle der Hohlkörperinnenwand. Das kapillar wirksame, dochtartige Material nimmt wiederum die kondensierte Flüssigkeit auf und transportiert diese zurück an eine Stelle der Heatpipe, an der Wärme zugeführt wird. Folglich bietet die Heatpipe einen geschlossenen Kühlkreislauf, dessen Flüssigkeit bzw. kondensierter Dampf nicht in das Innere des Gehäuses eintreten und mit den stromführenden Bauteilen des Beleuchtungssystems in Kontakt kommen kann.

In einer weiteren bevorzugten Ausgestaltung verjüngt sich das optische Lichtleitelement in Richtung zur Lichtquelle hin.

Diese Maßnahme hat den Vorteil, dass die Querschnittsfläche des ersten Endbereichs des optischen Lichtleitelements optimal an die Querschnittsfläche der Lichtquelle angepasst werden kann und folglich eine effiziente Einkopplung des Lichts in das optische Lichtleitelement ermöglicht wird. Ferner werden die Lichtstrahlen im optischen Lichtleitelement aufgrund seiner sich zum Lichtleitkabel hin aufweitenden Ausgestaltung aufgeweitet, so dass die Stirnfläche des Lichtleitkabels, das gewöhnlicherweise einen größeren Durchmesser als die Lichtquelle aufweist, optimal beleuchtet wird.

Das optische Lichtleitelement kann zwischen seinen beiden Endbereichen eine Einschnürung aufweisen.

In einer weiteren bevorzugten Ausgestaltung ist das optische Lichtleitelement kegelstumpfartig ausgebildet.

Diese Maßnahme ermöglicht eine vorteilhaft einfache Bauweise des sich verjüngenden optischen Lichtleitelements, die technisch einfach realisierbar ist. Wird bspw. zusätzlich auf die Außenfläche des optischen Lichtleitelements eine reflektierende Schicht zur Verringerung eines Intensitätsverlusts des Lichts im optischen Lichtleitelement aufgebracht, so kann dies besonders einfach erfolgen, da die Außenfläche des optischen Lichtleitelements glatt und ohne Wölbung ausgestaltet ist.

In einer weiteren bevorzugten Ausgestaltung ist das optische Lichtleitelement birnenförmig ausgebildet, wobei die optischen Lichtleitfasern im ersten Endbereich des optischen Lichtleitelements zur Lichtquelle hin gerichtet sind.

Diese-Maßnahme hat den Vorteil, dass die Lichtstrahlen, die von der annähernd punktförmigen Lichtquelle (LED) radial nach außen emittiert werden, mittels der zur Lichtquelle hinweisenden Lichtleitfasern optimal in das optische Lichtleitelement eingekoppelt werden können, so dass ein Lichtintensitätsverlust zwischen der Lichtquelle und dem optischen Lichtleitelement verringert wird.

In einer weiteren bevorzugten Ausgestaltung ist eine numerische Apertur des ernsten Endbereichs des optischen Lichtleitelements größer als eine numerische Apertur des zweiten Endbereichs des optischen Lichtleitelements.

Diese Maßnahme hat den Vorteil, dass das von der Lichtquelle emittierte, divergente Licht nach Durchlauf durch das optische Lichtleitelement in einen weniger divergenten Lichtstrahl umgewandelt wird, der dann ohne wesentlichen Intensitätsverlust in das optische Lichtleitkabel eingekoppelt werden kann.

In einer weiteren bevorzugten Ausgestaltung ist der erste Endbereich des optischen Lichtleitelements zumindest teilweise auf die Lichtquelle geklebt.

Diese Maßnahme hat den Vorteil, dass beim Einkoppeln des Lichts kein Lichtintensitätsverlust zwischen der Lichtquelle und dem optischen Lichtleitelement auftritt. Ferner kann eine zusätzliche Streuung der von der Lichtquelle emittierten Lichtstrahlen an zwischen der Lichtquelle und dem optischen Lichtleitelement befindlichen Luftmolekülen, reduziert werden, die eine noch größere Divergenz der Lichtstrahlen erzeugen würde. Ist die Lichtquelle geringfügig vom optischen Lichtleitelement beabstandet angeordnet, kann dieser Abstand mit dem Kleber gefüllt sein.

In einer weiteren bevorzugten Ausgestaltung weist das Beleuchtungssystem eine Buchse auf, durch die das proximale Ende des Lichtleitkabels in das Beleuchtungssystem eingeführt wird.

Diese Maßnahme hat den Vorteil, dass eine technisch einfach zu realisierende Möglichkeit bereitgestellt wird, das Lichtleitkabels reproduzierbar in das Beleuchtungssystem einzuführen und in die für eine Einkopplung des Lichts günstige Position zu bringen.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in den angegebenen Kombinationen, sondern auch in anderen Kombinationen oder in Alleiristellung einsetzbar sind, ohne den Rahmen der vorliegenden Erfindung zu verlassen.

Die Erfindung wird nachfolgend anhand einiger ausgewählter Ausführungsbeispiele im Zusammenhang mit der beiliegenden Zeichnung näher beschrieben und erläutert. Es zeigen:
- Fig. 1: eine schematische Darstellung eines Beleuchtungssystems;
- Fig. 2: eine detaillierte Darstellung des Beleuchtungssystems in Fig. 1;
- Fig. 3: eine Lichtquelle des Beleuchtungssystems in Fig. 2 in Alleinstellung;
- Fig. 4A-4D: verschiedene Ausführungsbeispiele eines optischen Lichtleitelements in Fig. 1; und
- Fig. 5: das Beleuchtungssystem in Fig. 1, während es mit einem Endoskop verbunden ist.

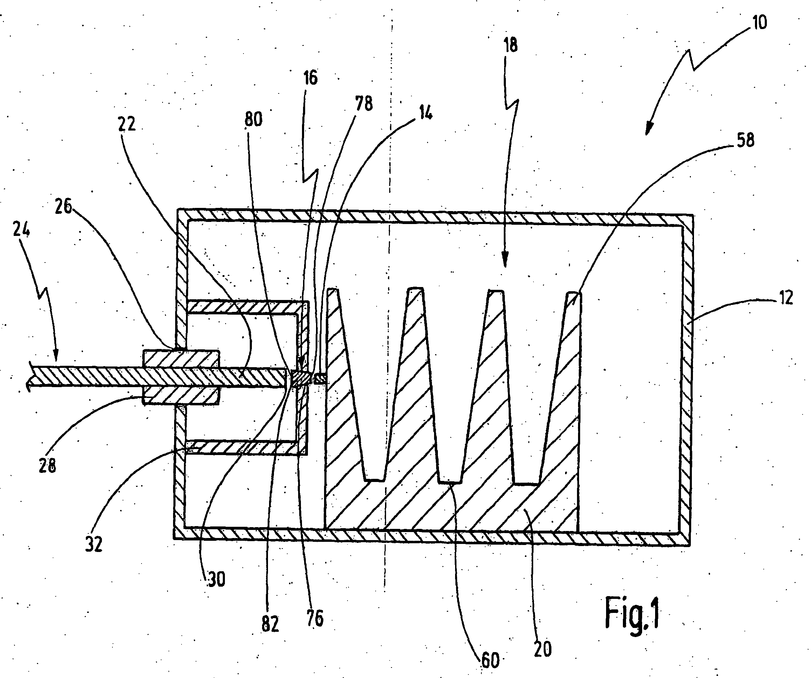

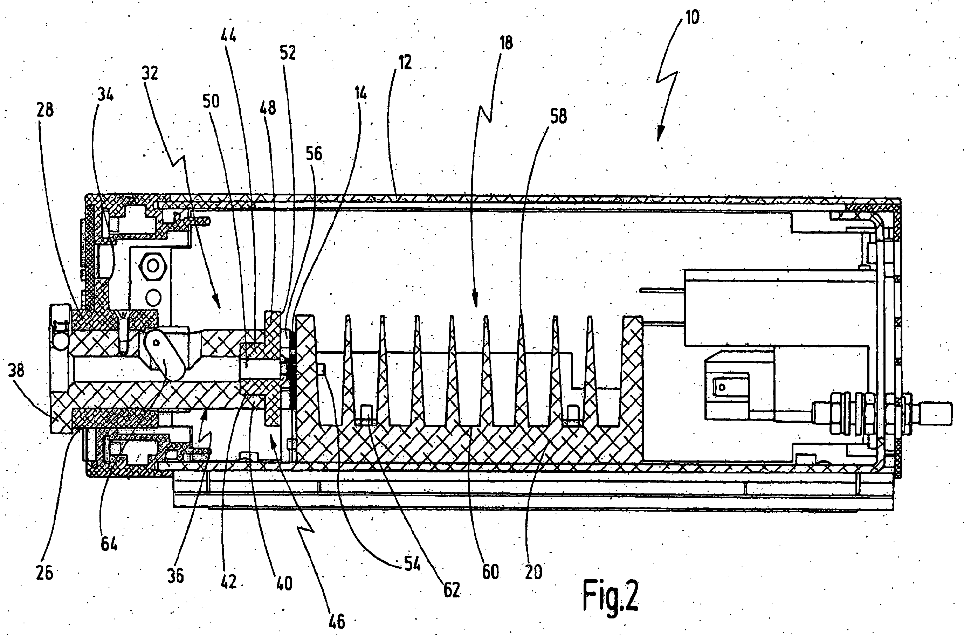

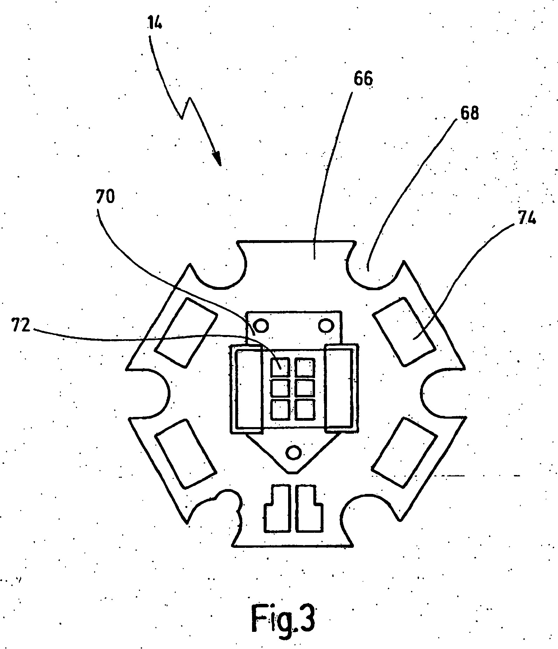

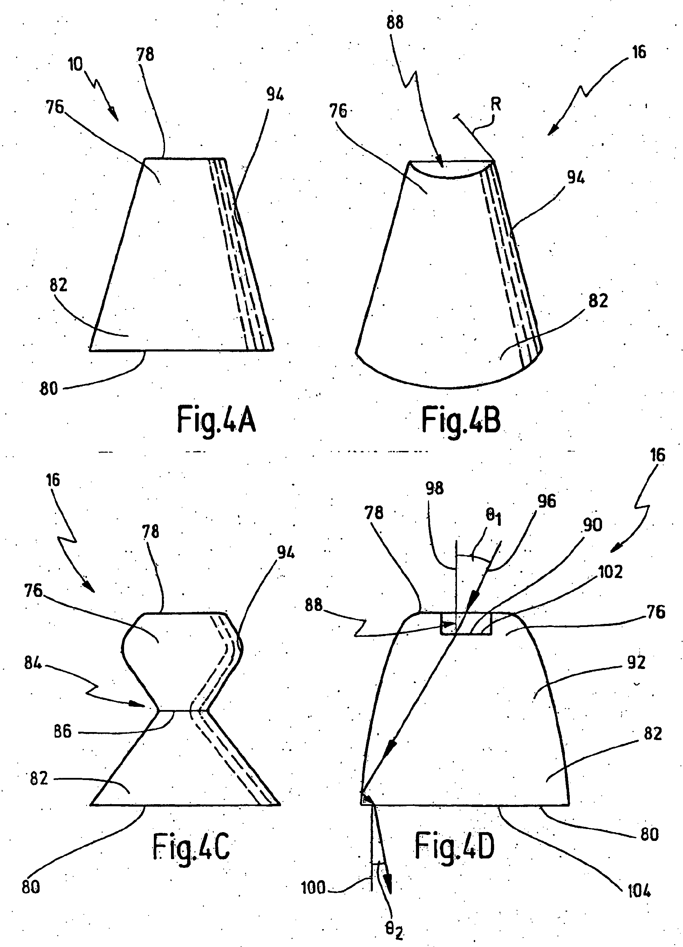

In Fig. 1 und 2 ist ein mit dem allgemeinen Bezugszeichen 10 versehenes Beleuchtungssystem dargestellt, wobei gleiche, gleichartige oder vergleichbare Bauteile dasselbe Bezugszeichen äufweisen. Weitere Einzelheiten des Beleuchtüngssystems 10 sind in Fig. 3 und Fig. 4A-4D dargestellt.

Das Beleuchtungssystem 10 wird bspw. in der Endoskopie oder Mikroskopie zur Ausleuchtung eines zu beobachtenden Bereiches verwendet.

Das Beleuchtungssystem 10 weist ein Gehäuse 12 auf, in dem eine Lichtquelle 14, ein optisches Lichtleitelement 16 und eine Kühlvorrichtung 18 angeordnet sind.

Die Lichtquelle 14 ist thermisch leitend mit der Kühlvorrichtung 18, d.h. mit einem wärmeleitfähigen Kühlkörper 20, verbunden, wobei unter einer thermisch leitenden Verbindung eine direkte oder indirekte thermische Kopplung der beiden Bauteile zu verstehen ist. Der Kühlkörper 20 ist an einer vom optischen Lichtleitelement 16 abgewandten Seite der Lichtquelle 14 angeordnet, wobei die Lichtquelle 14 vorzugsweise direkt am Kühlkörper 20 angeordnet ist, so dass von der Lichtquelle 14 erzeugte Wärme direkt an den Kühlkörper 20 abgegeben und von diesem abgeführt werden kann. Der Kühlkörper 20 ist ferner thermisch leitend mit dem Gehäuse 12 verbunden, so dass die vom Kühlkörper 20 aufgenommene Wärme an das Gehäuse 12 abgeführt werden kann. Der Kühlkörper 20 ist bezüglich seiner Abmessungen groß gegenüber der Lichtquelle 14 ausgebildet, so dass der Kühlkörper 20 effizient die von der Lichtquelle 14 erzeugte Wärme aufnehmen und abführen kann.

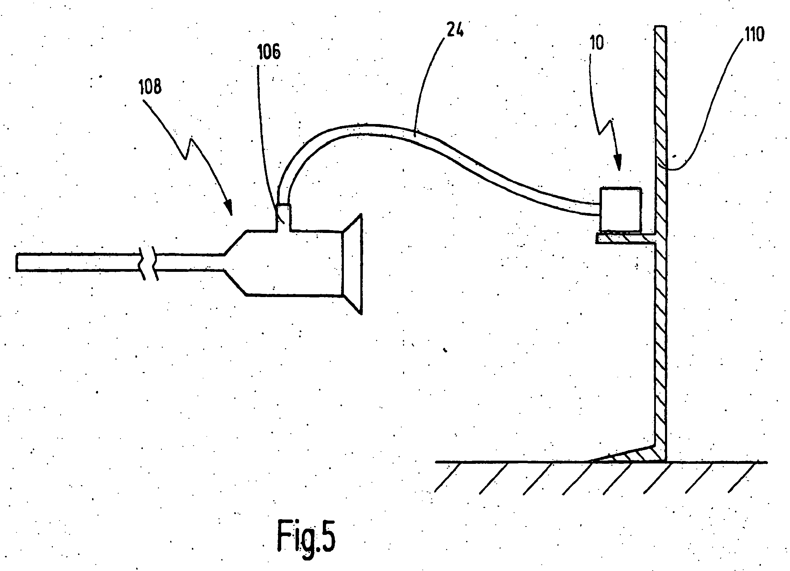

Ein proximales Ende 22 eines Lichtleitkabels 24, das bspw. mit einem Endoskop (vgl. Fig. 5) verbunden ist, ist durch eine in einer Öffnung 26 des Gehäuses 12 angeordneten Buchse 28 in das Gehäuse 12 einführbar bzw. das proximäle Ende 22 des Lichtleitkabels 24 kann in einem eingeführten Zustand fest im Beleuchtungssystem 10 angeordnet sein. Das proximale Ende 22 des Lichtleitkabels 24 kommt derart in dem Gehäuse 12 zu liegen, dass eine Stirnfläche 30 des proximalen Endes 22 des Lichtleitkabels 24 in Richtung der Lichtquelle 14 weist und nahe von dieser beabstandet angeordnet ist. Zwischen der Stirnfläche 30 des proximalen Endes 22 des Lichtleitkabels 24 und der Lichtquelle 14 ist das elektrisch isolierende optische Lichtleitelement 16 angeordnet, das durch ein elektrisch isolierendes Halteelement 32 in dem Gehäuse 12 gehalten wird.

Gemäß Fig. 2 ist in der Öffnung 26 die Buchse 28 angeordnet, um das proximale Ende 22 des Lichtleitkabels 24 in das Gehäuse 12 des Beleuchtungssystems 10 einzuführen. Die Buchse 28 ist als kurzes, zylindrisches Hohlrohr mit einer ringförmigen Verbreiterung 34 ausgebildet, die dazu dient, die Buchse 28 am Gehäuse 12 zu befestigen. In der Buchse 28 ist formschlüssig eine Fassung 36 angeordnet, die ebenfalls als zylindrisches Hohlrohr ausgebildet ist. Ein erster Endbereich 38 der Fassung 36, der außerhalb der Buchse 28, d.h. außerhalb des Gehäuses 12, angeordnet ist und durch den das proximale Ende 22 des Lichtleitkabels 24 in das Gehäuse 12 eingeführt wird, ist ringförmig verbreitert. Ein zweiter Endbereich 40 der Fassung 36 weist eine vollumfängliche Vertiefung 42 auf, deren Durchmesser größer als ein Innendurchmesser der Fassung 36 ausgebildet ist. In die Vertiefung 42 ist ein erster Abschnitt 44 einer Scheibe 46 formschlüssig aufgenommen. Ein zweiter ringförmiger Abschnitt 48 der Scheibe 46 weist einen größeren Außendurchmesser als der erste Abschnitt 44 der Scheibe 46 und als die Fassung 36 auf, so dass er über die Fassung 36 hinausragt. Die Scheibe 46 weist ferner einen zylindrischen Durchgang 50 auf, dessen Innendurchmesser etwa einem Innendurchmesser der Fassung 36 entspricht. In dem Durchgang 50 der Scheibe 46 ist das optische Lichtleitelement 14 (nicht dargestellt) angeordnet.

Das in Fig. 1 gezeigte Halteelement 32 ist hier als die Fassung 36 und die Scheibe 46 ausgebildet, wobei beide Bauteile aus einem elektrisch isolierenden Material, bspw. Kunststoff, hergestellt sind.

Die Lichtquelle 14 ist von einer Stirnseite 52 des zweiten Abschnitts 48 der Scheibe 46 beabstandet angeordnet und rückwärtig durch eine Schraube 54 mit dem Kühlkörper 20 verbunden, wobei die Schraube 54 bspw. als M3-Gewinde ausgebildet sein kann. Zu Stabilisationszwecken und zur Aufrechterhaltung eines Abstands zwischen der Lichtquelle 14 und des zweiten Abschnitts 48 der Scheibe 46 sind umfänglich verteilt Bolzen 56 durch den zweiten Endbereich 40 der Fassung 36, den zweiten Abschnitt 48 der Scheibe 46, die Lichtquelle 14 und die Kühlvorrichtung 16 gesteckt.

Die Kühlvorrichtung 18 ist vorzugsweise als passive Kühlung ausgebildet und arbeitet über eine Wärmekonvektion zwischen der Lichtquelle 14 und dem Kühlkörper 20. Sie weist zur Ableitung der von der Lichtquelle 14 erzeugten Wärme den wärmeleitenden Kühlkörper 20 auf, der auf der vom optischen Lichtleitelement 16 abgewandten Seite der Lichtquelle 14 angeordnet ist. Der Kühlkörper 20 weist ferner zur Erhöhung der Wärmeableitung abstehende Rippen 58 auf, die sich zu ihren freien Enden hin verjüngen. Die Rippen 58 sind derart voneinander beabstandet, dass in Zwischenflächen 60 Schrauben 62 angeordnet sind, mit denen der Kühlkörper 20 an dem Gehäuse 12 befestigt ist.

Die von der Lichtquelle 14 erzeugte Wärme wird über den direkten Kontakt zwischen der Lichtquelle 14 und dem Kühlkörper 20 sowie über die Schraube 54 an den Kühlkörper 20 abgegeben. Die dem Kühlkörper 20 zugeführte Wärme verteilt sich entlang des Kühlkörpers 20 und wird von diesem an das Gehäuse 12 abgegeben.

Die Kühlvorrichtung 18 kann ebenfalls als Heatpipe ausgebildet sein, wobei der Kühlkörper 20 hierzu als abgeschlossener Hohlkörper aus einem wärmeleitfähigen Material, bspw. Aluminium, ausgebildet ist. An einer Innenseite des Hohlkörpers ist ein kapillar wirksames, dochtartiges Material angeordnet. Der Hohlkörper ist ferner mit einer Flüssigkeit unter Eigendruck oder ggf. unter einem reduzierten Druck befüllt. Wird einer Oberfläche der Heatpipe Wärme von der Lichtquelle 14 zugeführt, so beginnt die in einem Inneren der Heatpipe befindliche Flüssigkeit zu sieden und geht unter Aufnahme von Wärmeenergie in Dampf über. Dieser Dampf verteilt sich in dem Hohlkörper und kondensiert unter Wärmeabgabe an einer kälteren Stelle einer Innenwand der Heatpipe. Das kapillar wirksame, dochtartige Material nimmt wiederum die kondensierte Flüssigkeit auf und transportiert diese zurück an eine Stelle der Heatpipe, an der Wärme zugeführt wird. Die Heatpipe bildet daher einen geschlossenen Kühlkreislauf, mit dem das Beleuchtungssystem 10 effizient gekühlt werden kann.

Das proximale Ende 22 des Lichtleitkabels 24 kann durch einen Fixiermechanismus in der Fassung 36 gehalten werden. Hierzu ist ein Verschlusshebel 64 an der Fassung 36 angeordnet, mittels dem bspw. das proximale Ende 22 des Lichtleitkabels 24 eingeklemmt werden kann.

Die Lichtquelle 14 ist bspw., wie in Fig. 3 dargestellt, als herkömmliche OSTAR^{®}-Lighting Lichtquelle der Firma Osram ausgebildet. Diese Lichtquelle weist einen flachen, hexagonalen Aluminiummetallkörper 66 auf, an dessen jeweiligen Ecken je .eine Aussparung 68 ausgebildet ist. Durch diese Aussparungen 68 sind die Bolzen 56 durchsteckbar. Auf dem Metallkörper 66 sind sechs auf einer Keramikscheibe 70 montierte GaN-LEDs 72 angeordnet, die Licht im weißen Spektralbereich abstrahlen. Die LEDs 72 sind über entsprechende Kontakte 74 auf dem Metallkörper 66 elektrisch kontaktierbar.

Fig. 4A-4D zeigen verschieden Ausgestaltungen des optischen Lichtleitelements 16. Das optische Lichtleitelement 16 weist einen ersten Endbereich 76 auf, dessen Querschnittsfläche 78 kleiner als eine Querschnittsfläche 80 eines zweiten Endbereichs 82 des optischen Lichtleitelements 16 ausgebildet ist. Das optische Lichtleitelement 16 ist derart in dem Beleuchtungssystem 10 aufgenommen, dass der erste Endbereich 76 zur Lichtquelle 16 hinweist, während der zweite Endbereich 82 zur Stirnfläche 30 des proximalen Endes 22 des Lichtleitkabels 24 gerichtet ist (siehe Fig. 1).

Ferner kann das optische Lichtleitelement 16 kegelstumpfartig (siehe Fig. 4A, 4B) oder auch birnenförmig (siehe Fig. 4C, 4D) ausgebildet sein, wobei eine beliebige Querschnittsfläche des optischen Lichtleitelements 16 zwischen den beiden Endbereichen 76, 82 bspw. parabolisch, elliptisch, hyperbolisch, kreisförmig oder konisch ist. Vorzugsweise ist das optische Lichtleitelement 16 als Compound Elliptical Concentrator (CEC) oder als Compound Hyperbolic Concentrator (CHC) ausgebildet.

Das optische Lichtleitelement 16 verjüngt sich vom zweiten Endbereich 82 zum ersten Endbereich 76. Ferner kann das optische Lichtleitelement 16 eine Einschnürung 84 zwischen dem ersten Endbereich 76 und dem zweiten Endbereich 82 aufweisen (siehe Fig. 4C). Eine Querschnittsfläche 86 im Bereich der Einschnürung 84 ist kleiner als die Querschnittsfläche 80 des zweiten Endbereichs 82 sowie kleiner bzw. größer als die Querschnittsfläche 78 des ersten Endbereichs 70 ausgebildet.

Ferner kann der erste Endbereich 76 des optischen Lichtleitelements 16 eine Vertiefung 88 aufweisen, in der die LED 72 zumindest teilweise aufgenommen werden kann. Die Vertiefung 88 kann bspw. in Form einer Oberfläche eines Kugelsegments (mit Radius R, siehe Fig. 4B), zylinderförmig oder auch würfelförmig (siehe Fig. 4D) zur zumindest teilweisen Aufnahme der LED 72 ausgebildet sein, wobei eine Querschnittsfläche 90 der Vertiefung 88 kleiner als die Querschnittsfläche 78 des ersten Endbereichs 76 des optischen Lichtleitelements 16 ausgebildet sein kann. Der zweite Endbereich 82 des optischen Lichtleitelements 16 kann in Richtung zum proximalen Ende 22 des Lichtleitkabels 24 hingewölbt sein (siehe Fig. 4B), wobei er bspw. wabenartig oder glatt geformt sein kann.

Das optische Lichtleitelement 16 ist vorzugsweise als Glaskörper 92, insbesondere als Linse, ausgebildet (siehe Fig. 4D). In einer alternativen Ausgestaltung ist das optische Lichtleitelement 16 aus optischen Lichtleitfasern 94 aufgebaut. Die optischen Lichtleitfasern 94 können im ersten Endbereich 76 des optischen Lichtleitelements 16 zur Lichtquelle 14 hin gerichtet sein. Ferner können sich die optischen Lichtleitfasern 94 zum ersten Endbereich 76 hin verjüngen. Wie in Fig. 4D dargestellt, ist eine numerische Apertur des ersten Endbereichs 76 des optischen Lichtleitelements 16 größer als eine numerische Apertur des zweiten Endbereichs 82 des optischen Lichtleitelements 16 ausgebildet, so dass ein Eintrittswinkel Θ₁ eines Lichtstrahls 96, der von der Lichtquelle 14 emittiert wird, größer als ein Austrittswinkel Θ₂ ist. Hierbei ist Θ₁, Θ₂ ein Zwischenwinkel zwischen einer Normalen 98, 100 und einer Oberfläche 102, 104 des ersten Endbereichs 76 bzw. des zweiten Endbereichs 82.

Das optische Lichtleitelement 16 ist, da es aus Glas bzw. aus Glasfasern ausgebildet ist, elektrisch isolierend, so dass keine Ströme von der mit Spannung beaufschlagbaren Lichtquelle 14 auf das optische Lichtleitelement 16 und das proximale Ende 22 des Lichtleitkabels 24 bzw. das Gehäuse 12 übertreten können.

Das optische Lichtleitelement 16 kann geringfügig beabstandet von der Lichtquelle 14 angeordnet sein, wobei der Abstand vorzugsweise 2/10 mm bis 4/10 mm betragen kann. Das optische Lichtleitelement 16 kann ebenfalls mittels eines transmittierenden Klebers zumindest teilweise auf die Lichtquelle 14 aufgeklebt sein, um eine Lichtstreuung an Luftmolekülen zwischen der Lichtquelle 14 und dem optischen Lichtleitelement 16 zu verringern. Hierbei kann der Abstand zwischen der Lichtquelle und dem optischen Lichtleitelement mit dem Kleber gefüllt sein.

Wie in Fig. 5 dargestellt, wird das Beleuchtungssystems 10 während eines Betriebs über das Lichtleitkabel 24 bspw. mit einem Anschluss 106 des Endoskops 108 verbunden, um das von der Lichtquelle 14 emittierte Licht über das Lichtleitkabel 24 und eine Lichtleitoptik, die in dem Endoskop 108 aufgenommen ist (nicht dargestellt), zu dem zu beleuchtenden Bereich zu führen. Während des Betriebs kann das Beleuchtungssystems 10 auf einer Halterung 110, bspw. einem Rack, angeordnet sein.

## Patentansprüche

1. Beleuchtungssystem zum Erzeugen von Licht und zum Einkoppeln des Lichts in ein proximales Ende (22) eines Lichtleitkabels (24) einer Beobachtungsvorrichtung für die Endoskopie oder Mikroskopie, mit einem Lichtleitkabel (24), mit einer zumindest eine LED (72) aufweisenden Lichtquelle (14), mit einem als Glaskörper (92) ausgebildeten oder aus Lichtleitfasern (94) aufgebauten und elektrisch isolierenden optischen Lichtleitelement (16) zwischen der Lichtquelle (14) und dem proximalen Ende (22) des Lichtleitkabels (24) zum Einleiten des von der Lichtquelle (14) emittierten Lichts in das proximale Ende (22) des Lichtleitkabels (24), wobei ein erster Endbereich (76) des optischen Lichtleitelements (16) auf einer der Lichtquelle (14) zugewandten Seite eine kleinere Querschnittsfläche (78) als ein zweiter Endbereich (82) des optischen Lichtleitelements (16) auf einer dem proximalen Ende (22) des Lichtleitkabels (24) zugewandten Seite aufweist, und mit einer Kühlvorrichtung (18), die zumindest einen Kühlkörper (20) aufweist und die mit der Lichtquelle (14) thermisch leitend verbunden ist, um von der Lichtquelle (14) erzeugte Wärme aus dem Beleuchtungssystem (10) abzuführen, der Kühlkörper (20) vollständig auf einer dem optischen Lichtieltelememt (16) abgewandten Seite der Lichtquelle (14) angeordnet ist, wobei das Lichtleitelement (16) von der Lichtquelle (14) geringfügig beabstandet angeordnet ist **dadurch gekennzeichnet, dass** das Lichtleitkabel (24) zwischen dem Lichtleitelement (16) und einer mit dem Lichtleitkabel (24) zu verbindenden Beobachtungsvorrichtung angeordnet ist, und dass das Lichtieltelement (16) in einem elektrisch isolierenden Halteelement (32) angeordnet ist.

2. Beleuchtungssystem nach Anspruch 1, **dadurch gekennzeichnet, dass** die Lichtquelle (14); das optische Lichtleitelement (16), das proximale Ende (22) des Lichtleitkabels (24) und der Kühlkörper (20) in einem gemeinsamen Gehäuse (12) angeordnet sind.

3. Beleuchtungssystem nach Anspruch 2, **dadurch gekennzeichnet, dass** der Kühlkörper (20) thermisch leitend, insbesondere direkt thermisch leitend, mit dem Gehäuse (12) verbunden ist.

4. Beleuchtungssystem nach einem der Ansprüche 1 bis 3, **dadurch gekennzeichnet, dass** der Kühlkörper (20) als passive Kühlung, insbesondere als Heatpipe, ausgebildet ist.

5. Beleuchtungssystem nach einem der Ansprüche 1 bis 4, **dadurch gekennzeichnet, dass** das optische Lichtleitelement (16) als Linse ausgebildet ist.

6. Beleuchtungssystem nach einem der Ansprüche 1 bis 5, **dadurch gekennzeichnet, dass** sich das optische Lichtleitelement (16) in Richtung zur Lichtquelle (14) hin verjüngt.

7. Beleuchtungssystem nach einem der Ansprüche 1 bis 6, **dadurch gekennzeichnet, dass** das optische Lichtleitelement (16) kegelstumpfartig ausgebildet ist.

8. Beleuchtungssystem nach einem der Ansprüche 1 bis 7, **dadurch gekennzeichnet, dass** das optische Lichtleitelement (16) birnenförmig ausgebildet ist, wobei die optischen Lichtleitfasern (94) im ersten Endbereich (76) des optischen Lichtleitelements (16) zur Lichtquelle (14) hin gerichtet sind.

9. Beleuchtungssystem nach einem der Ansprüche 1 bis 8, **dadurch gekennzeichnet, dass** eine numerische Apertur des ersten Endbereichs (76) des optischen Lichtleitelements (16) größer als eine numerische Apertur des zweiten Endbereichs (82) des optischen Lichtleitelements (16) ist.

10. Beleuchtungssystem nach einem der Ansprüche 1 bis 9, **dadurch gekennzeichnet, dass** der erste Endbereich (76) des optischen Lichtleitelements (16) zumindest teilweise auf die Lichtquelle (14) geklebt ist.

11. Beleuchtungssystem nach einem der Ansprüche 1 bis 10, **dadurch gekennzeichnet, dass** das Beleuchtungssystem (10) eine Buchse (28) aufweist, durch die das proximale Ende (22) des Lichtleitkabels (24) in das Beleuchtungssystem (10) eingeführt wird.

## Claims

1. An illumination system for producing light and for launching the light into a proximal end (22) of an optical cable (24) of an observation apparatus for endoscopy or microscopy, comprising an optical cable (24), a light source (14) which has at least one LED (72), an optical light guiding element (16) between the light source (14) and the proximal end (22) of the optical cable (24) for introducing the light emitted by the light source (14) into the proximal end (22) of the optical cable (24), the optical light guiding element (16) being configured as a glass body (92) or constructed from optical fibres (94) and electrically insulating, wherein a first end region (76) of the optical light guiding element (16) on a side facing the light source (14) has a smaller cross-sectional area (78) than a second end region (82) of the optical light guiding element (16) on a side facing the proximal end (22) of the optical cable (24), and further comprising a cooling apparatus (18) which has at least one cooling body (20) and which is thermally conductively connected to the light source (14) in order to remove heat produced by the light source (14) from the illumination system (10), the cooling body (20) being arranged completely on a side of the light source (14) which is remote from the optical light guiding element (16), the light guiding element (16) being slightly spaced apart from the light source (14), **characterized in that** the optical cable (24) is arranged between the light guiding element (16) and an observation apparatus to be connected with the optical cable (24), and that the light guiding element (16) is arranged in an electrically insulating holder element (32).

2. The illumination system of Claim 1, **characterized in that** the light source (14), the optical light guiding element (16), the proximal end (22) of the optical cable (24) and the cooling body (20) are arranged in a common housing (12).

3. The illumination system of Claim 2, **characterized in that** the cooling body (20) is connected in thermally conductive fashion, particularly in directly thermally conductive fashion, to the housing (12).

4. The illumination system of anyone of Claims 1 through 3, **characterized in that** the cooling body (20) is configured as passive cooling, particularly in the form of a heatpipe.

5. The illumination system of anyone of Claims 1 through 4, **characterized in that** the optical light guiding element (16) is configured as a lens.

6. The illumination system of anyone of Claims 1 through 5, **characterized in that** the optical light guiding element (16) tapers in direction to the light source (14).

7. The illumination system of anyone of Claims 1 through 6, **characterized in that** the optical light guiding element (16) is configured as a truncated cone.

8. The illumination system of anyone of Claims 1 through 7, **characterized in that** the optical light guiding element (16) is configured in pear-shaped form, with the optical fibres (94) in the first end region (76) of the optical light guiding element (16) being directed towards the light source (14).

9. The illumination system of anyone of Claims 1 through 8, **characterized in that** a numerical aperture of the first end region (76) of the optical light guiding element (16) is larger than a numerical aperture of the second end region (82) of the optical light guiding element (16).

10. The illumination system of anyone of Claims 1 through 9, **characterized in that** the first end region (76) of the optical light guiding element (16) is at least partially glued onto the light source (14).

11. The illumination system of anyone of Claims 1 through 10, **characterized in that** the illumination system (10) has a socket (28) through which the proximal end (22) of the optical cable (24) is introduced into the illumination system (10).

## Revendications

1. Système d'éclairage pour produire de la lumière et pour injecter la lumière dans une extrémité proximale (22) d'un câble de guidage de lumière (24) d'un dispositif d'observation pour l'endoscopie ou la microscopie, avec un câble de guidage de lumière (24), avec une source de lumière (14) présentant au moins une DEL (72), avec un élément de guidage de lumière (16) optique isolant électriquement, réalisé en tant que corps en verre (92) ou formé par des fibres optiques (94) entre la source de lumière (14) et l'extrémité proximale (22) du câble de guidage de lumière (24), pour introduite la lumière émise par la source de lumière (14) dans l'extrémité proximale (22) du câble de guidage de lumière (24), dans lequel une première zone d'extrémité (76) de l'élément de guidage de lumière (16) optique présente, sur un côté tourné vers la source de lumière (14), une surface de section transversale plus petite qu'une deuxième zone d'extrémité (82) de l'élément de guidage de lumière (16) optique sur un côté tourné vers l'extrémité proximale (22) du câble de guidage de lumière (24), et avec un dispositif de refroidissement (18) qui présente au moins un corps de refroidissement (20) et qui est relié de manière thermoconductrice à la source de lumière (14) afin d'évacuer de la chaleur, produite par la source de lumière (14), du système d'éclairage (10), le corps de refroidissement (20) est entièrement disposé sur un côté de la source de lumière (14) tournant le dos à l'élément de guidage de lumière (16) optique, l'élément de guidage de lumière (16) étant disposé de manière légèrement espacée par rapport à la source de lumière (14), **caractérisé en ce que** le câble de guidage de lumière (24) est disposé entre l'élément de guidage de lumière (16) et un dispositif d'observation à relier au câble de guidage de lumière (24), et que l'élément de guidage de lumière (16) est disposé dans un élément de retenue (32) isolant électriquement.

2. Système d'éclairage selon la revendication 1, **caractérisé en ce que** la source de lumière (14), l'élément de guidage de lumière (16) optique, l'extrémité proximale (22) du câble de guidage de lumière (24) et le corps de refroidissement (20) sont disposés dans un boîtier (12) commun.

3. Système d'éclairage selon la revendication 2, **caractérisé en ce que** le corps de refroidissement (20) est relié de manière thermoconductrice, en particulier de manière directement thermoconductrice, au boîtier (12).

4. Système d'éclairage selon l'une des revendications 1 à 3, **caractérisé en ce que** le corps de refroidissement (20) est réalisé en tant que refroidissement passif, en particulier en tant que caloduc.

5. Système d'éclairage selon l'une des revendications 1 à 4, **caractérisé en ce que** l'élément de guidage de lumière (16) optique est réalisé en tant que lentille.

6. Système d'éclairage selon l'une des revendications 1 à 5, **caractérisé en ce que** l'élément de guidage de lumière (16) optique s'amenuise en direction de la source de lumière (14).

7. Système d'éclairage selon l'une des revendications 1 à 6, **caractérisé en ce que** l'élément de guidage de lumière (16) optique est réalisé de manière tronconique.

8. Système d'éclairage selon l'une des revendications 1 à 7, **caractérisé en ce que** l'élément de guidage de lumière (16) optique est réalisé en forme de bulbe, dans lequel les fibres optiques (94) optiques dans la première zone d'extrémité (76) de l'élément de guidage de lumière (16) optique sont orientées vers la source de lumière (14).

9. Système d'éclairage selon l'une des revendications 1 à 8, **caractérisé en ce qu'**une ouverture numérique de la première zone d'extrémité (76) de l'élément de guidage de lumière (16) optique est plus grande qu'une ouverture numérique de la deuxième zone d'extrémité (82) de l'élément de guidage de lumière (16) optique.

10. Système d'éclairage selon l'une des revendications 1 à 9, **caractérisé en ce que** la première zone d'extrémité (76) de l'élément de guidage de lumière (16) optique est au moins partiellement collée sur la source de lumière (14).

11. Système d'éclairage selon l'une des revendications 1 à 10, **caractérisé en ce que** le système d'éclairage (10) présente une douille (28), grâce à laquelle l'extrémité proximale (22) du câble de guidage de lumière (24) est insérée dans le système d'éclairage (10).
